(19) Europäisches Patentamt
European Patent Office
Office européen des brevets

(11) EP 2 119 428 B1

(12) **EUROPEAN PATENT SPECIFICATION**

(45) Date of publication and mention of the grant of the patent:
**02.11.2016 Bulletin 2016/44**

(21) Application number: **08702844.5**

(22) Date of filing: **30.01.2008**

(51) Int Cl.:
***A61K 8/34*** *(2006.01)* *A61Q 5/06* *(2006.01)*

(86) International application number:
**PCT/JP2008/000112**

(87) International publication number:
**WO 2008/093500 (07.08.2008 Gazette 2008/32)**

(54) **Use and method for reducing hair frizz**

Verwendung und Verfahren zur Verminderung der Haarkrause

Utilisation et procédé destiné à la réduction des cheveux frisés

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.01.2007 JP 2007019045**

(43) Date of publication of application:
**18.11.2009 Bulletin 2009/47**

(73) Proprietor: **Kao Corporation**
**Chuo-Ku**
**Tokyo 103-8210 (JP)**

(72) Inventors:
- **NAGASE, Shinobu**
  **Tokyo 131-8501 (JP)**
- **YAMAGUCHI, Masakazu**
  **Tokyo 131-8501 (JP)**

(74) Representative: **Hoffmann Eitle**
**Patent- und Rechtsanwälte PartmbB**
**Arabellastraße 30**
**81925 München (DE)**

(56) References cited:

| | |
|---|---|
| **WO-A1-01/91705** | **WO-A1-2005/084620** |
| **WO-A2-01/26604** | **JP-A- 07 048 230** |
| **JP-A- 10 072 321** | **JP-A- 10 316 542** |
| **JP-A- 2002 173 449** | **JP-A- 2002 326 913** |
| **JP-A- 2003 063 934** | **JP-A- 2003 183 132** |
| **JP-A- 2003 221 313** | **JP-A- 2004 091 455** |
| **JP-A- 2005 330 214** | **JP-A- 2005 330 216** |
| **JP-A- 2006 347 997** | |

- **Ernest W. Flick: "Cosmetic and toiletry formulations", 2001, Noyes Publications, XP002689691, ISBN: 0-8155-1454-9 vol. 8, page 271,273, * the whole document ***
- **M.J. Fresno Contreras ET AL: "Viscosity and temperature relationship in ethanol/water mixtures gelified with Carbopol Ultrez(TM) 10", Il Farmaco, 1 January 2001 (2001-01-01), pages 443-445, XP055048599, Retrieved from the Internet: URL:http://ac.els-cdn.com/S0014827X01010588/1-s2.0-S0014827X01010588-main.pdf?_tid=d2f1e75c-5590-11e2-b85b-00000aab0f02&acdnat=1357209307_02e6ae18145333b8fae23c395144b3 cd [retrieved on 2013-01-03]**
- **Anonymous: "NATROSOL - Hydroxyethylcellulose - A Nonionic Water-Soluble Polymer", Ashland , 1999, XP002689692, Retrieved from the Internet: URL:http://www.ashland.com/Ashland/Static/Documents/AAFI/PRO-250-11G_Natrosol11G.pdf [retrieved on 2013-01-02]**
- **Anonymous: "Got Hair Frizz? Care Product Ingredients to Watch Out For", Emtage Hair , 18 November 2011 (2011-11-18), XP002689693, Retrieved from the Internet: URL:http://emtagehair.wordpress.com/2011/11/18/got-hair-frizz-hair-care-product-ingredients-to-watch-out-for/ [retrieved on 2013-01-02]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **Anonymous: "Relaxing Hair Balm & Defrizzer S-X0005 - Formulation Details", The Lubrizol Corporation , 23 October 2007 (2007-10-23), XP002689694, Retrieved from the Internet: URL:http://www.lubrizol.com/PersonalCare/S-X0005-Relaxing-Hair-Balm-Defrizzer.pdf [retrieved on 2013-01-02]**
- **Anonymous: "Enriched Hair Glosser S-X0015", The Lubrizol Corporation , 23 October 2007 (2007-10-23), XP002689695, Retrieved from the Internet: URL:http://www.lubrizol.com/PersonalCare/S-X0015-Enriched-Hair-Glosser.pdf [retrieved on 2013-01-02]**

## Description

### Technical Field

The present invention relates to a hair frizziness reducing agent.

### Background Art

It is widely known that as a phenomenon associated with aging, hair thinning occurs due to decrease in hair diameter or reduction in the density of terminal hair or grey hair increases. The present inventors specifically studied the aging phenomenon of hair and have found that in addition to the thinning of hair and increase in gray hair, a percentage of meandering hair (frizzy hair) increases. They have also found that there occurs a difference in cell structure inside the hair between such frizzy hair and straight hair. An increase in such frizzy hair is presumed to cause deterioration in both manageability and luster of the hair, leading to hair troubles of not-so-young people.

Although some proposals such as treatment for enhancing strength/body of hair (for example, Patent Document 1) and treatment for providing hair with volume (for example, Patent Document 2) have been made as a measure for overcoming hair troubles accompanying the aging such as decrease in hair diameter and reduction in the density of terminal hair, they do not produce a satisfactory effect. Straight perming has also been performed widely for straightening of frizzy hair, but it is not satisfactory because the hair itself is inevitably damaged thereby.

[Patent Document 1] JP-A-6-305942

[Patent Document 2] JP-A-2000-38323

Ernest W. Flick, "Cosmetic and toiletry formulations", Vol,8, pages 271 and 273 (Noyes Publications 2001) describe a hand cleaner and a hand sanitizing composition comprising water, an alcohol and a thickening agent.

Fresno et al., Il Farmaco, 56 (2001) (pages 443-445) examine the viscosity of ethanol/water mixtures gelified with Carbopol Ultrez™ 10 and the temperature dependency thereof.

WO 2001/91705 describe a is a hair conditioning composition comprising: (1) a thickening agent selected from the group consisting of an acrylic acid/alkyl acrylate copolymer, an acrylates copolymer, and a crosslinked polymer; (2) a frizz control agent selected from the group consisting of PEG-modified glycerides, PEG-modified glyceryl fatty acid esters, dimethicone copolyols, and mixture thereof; and (3) an aqueous carrier. The aqueous carrier may optionally comprise an alcohol.

WO 2005/084620 relates to a hair fixative composition comprising a canonically modified hydrolysed starch and especially canonically modified hydrolysed starches, hydrolysed to the extent of having a dextrose equivalent of 1 to 6.

WO 2001/26604 describes a method, which comprises applying to scalp a treatment lotion, based on a protein component, a medical component and a fatty component. The protein component is based on natural milk, the medical component is mainly herbal and the fatty component is based on vegetable fats. The new method is said to allow relieving headaches, sensitivity to scalp pains, sense of dryness, to impart pleasant feeling to the scalp, to improve hair flexibility, to remove dandruff, and to be suitable for treating seborrhea and other hair problems.

### [Disclosure of the Invention]

The present invention relates to the use of an agent, which is applied to the scalp as is and left on the scalp without washing away, for reducing the frizziness of the hair which has grown after the application of the agent,
wherein the agent comprises a lower alcohol in an amount of 5 mass% to 80 mass%, water, and a hair growing/nourishing component; and has a viscosity of from 1 to 90,000 mPa*s, measured by a Brookfield viscometer at 23°C;
wherein the lower alcohol is at least one selected from ethanol, 1- propanol, 2-propanol, n-butanol, 2-phenoxyethanol, benzyl alcohol, 2- phenylethanol, 2-benzyloxyethanol, triethylene glycol, 1,3-butylene glycol, propylene glycol, hexylene glycol, and diethylene glycol; and
wherein the hair growing/nourishing component is at least one selected from blood circulation enhancers, follicle activators, anti-androgen agents, and potassium ion channel openers.

In another aspect, the present invention is directed to a method for reducing the frizziness of hair which has grown after the application of the above defined agent, wherein the method comprises application of the agent to the scalp as is an leaving it on the scalp without washing away; the agent being periodically applied to the scalp once or more every two days for 2 months or more.

[Mode for Carrying out the Invention]

The present invention relates to the use of a hair frizziness reducing agent capable of reducing hair frizziness which has occurred with aging. It should be noted that the term "hair frizziness" as used herein means frizziness that increases with the advance of age.

The present inventors have found that hair frizziness can be reduced by applying, to a scalp, a composition containing a lower alcohol and water and having a predetermined viscosity and then leaving it without washing it away.

<Lower alcohol>

The lower alcohol contained in the hair frizziness reducing agent is at least one selected from ethanol, 1-propanol, 2-propanol, and n-butanol; 2-phenoxyethanol, benzyl alcohol, 2-phenylethanol, 2-benzyloxyethanol; triethylene glycol, 1,3-butylene glycol, propylene glycol, hexylene glycol, and diethylene glycol.

These lower alcohols may be used either singly or in combination. The content of the lower alcohol is from 5 to 80 mass%, preferably from 5 to 70 mass%, more preferably from 5 to 65 mass% in the hair frizziness reducing agent of the present invention from the standpoint of solubilization of organic components.

<Water>

The water content in the hair frizziness reducing agent of the present invention is preferably from 20 to 99 mass%, more preferably from 35 to 80 mass%, even more preferably from 40 to 70 mass% from the standpoint of solubilization of organic components.

The viscosity of the hair frizziness reducing agent of the present invention as measured by a Brookfield viscometer falls within a range of preferably from 1 to 90,000 mPa·s, more preferably from 2 to 50,000 mPa·s, even more preferably from 3 to 10,000 mPa·s because the viscosity within the above range is expected to produce a satisfactory effect by enabling an adequate amount of the hair frizziness reducing agent to remain on the scalp and at the same time, it facilitates uniform application of the agent to the hair.

The viscosity of the hair frizziness reducing agent can be adjusted to a desired range by using a surfactant or a polymeric thickener.

<Surfactant>

The hair frizziness reducing agent of the present invention can be adjusted to a desired viscosity with a surfactant such as nonionic surfactant, amphoteric surfactant, cationic surfactant, or anionic surfactant.

Examples of the nonionic surfactant include polyoxyalkylene alkyl ethers, polyoxyalkylene alkenyl ethers, higher fatty acid sucrose esters, polyglycerin fatty acid esters, higher fatty acid mono- or diethanolamides, polyoxyethylene hydrogenated castor oils, polyoxyethylene sorbitan fatty acid esters, polyoxyethylene sorbitol fatty acid esters, alkyl saccharide surfactants, alkyl amine oxides, and alkylamidoamine oxides. Of these, polyoxyalkylene alkyl ethers and polyoxyethylene hydrogenated castor oils are preferred, with polyoxyethylene alkyl ethers being more preferred. The polyoxyethylene alkyl ethers are preferably represented by the following formula:

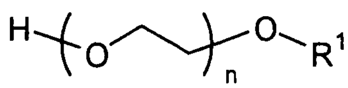

[wherein, $R^1$ represents a linear or branched alkyl group having from 8 to 18 carbon atoms and n stands for the number of from 1 to 25].

Examples of the amphoteric surfactant include imidazoline, carbobetaine, amidobetaine, sulfobetaine, hydroxysulfobetaine, and amidosulfobetaine surfactants. Of these, betaine surfactants such as alkyldimethylaminoacetic acid betaines and fatty acid amidopropyl betaines are preferred, with fatty acid amidopropyl betaines being more preferred. The fatty acid amidopropyl betaines preferably have an acyl group having from 8 to 18, more preferably from 10 to 16 carbon atoms, with lauramidopropyl betaine, palm kernel amidopropyl betaine and cocamidopropyl betaine being even more preferred.

The cationic surfactant is preferably a mono- or di(long chain)alkyl quaternary ammonium salt having 16 or more carbon atoms. Specific examples of it include stearyltrimethylammonium chloride, arachyltrimethylammonium chloride, behenyltrimethylammonium chloride, and alkylbenzalkonium chloride. Of these, stearyltrimethylammonium chloride and behenyltrimethylammonium chloride are preferred.

Examples of the anionic surfactant include alkylbenzene sulfonates, alkyl or alkenyl ether sulfates, alkyl or

alkenyl sulfates, olefin sulfonates, alkane sulfonates, saturated or unsaturated fatty acid salts, alkyl or alkenyl ether carboxylates, α-sulfone fatty acid salts, N-acylamino acid surfactants, mono- or di-phosphoric diester surfactants, and sulfosuccinates. Examples of the counterion for the anionic residue of these anionic surfactants include alkali metal ions such as sodium ion and potassium ion; alkaline earth metal ions such as calcium ion and magnesium ion; ammonium ion; and alkanolamines (e.g., monoethanolamine, diethanolamine, triethanolamine, and triisopropanolamine) having from 1 to 3 alkanol groups with 2 or 3 carbon atoms.

These surfactants may be used either singly or in combination. The total content of them in the hair frizziness reducing agent of the present invention is preferably from 0.01 to 20 mass%, more preferably from 0.05 to 15 mass%, even more preferably from 0.1 to 10 mass% from the standpoint of providing the hair with good feel.

<Polymeric thickener>

The hair frizziness reducing agent of the present invention is preferably adjusted to have a desired viscosity with a synthetic polymer or a natural polymer. Examples of the natural polymer or synthetic polymer usable for this purpose include dimethyl diallyl ammonium chloride/acrylamide copolymers (e.g., "Merquat 100" and "Merquat 550", each product of Nalco), vinylpyrrolidone/dimethyl aminoethyl methacrylate copolymers (e.g., "Copolymer 845", "Copolymer 937", and "Copolymer 958", each product of ISP), acrylic acid/alkyl methacrylate copolymers (e.g., "Pemulen TR-1" and "Pemulen TR-2", each product of Noveon), o-[2-hydroxy-3-(lauryldimethylammonio)propyl]hydroxyethyl cellulose chlorides (e.g., "Polymer JR-125", "Polymer JR-30M" and "Polymer JR 400", each product of The Dow Chemical Company and "Leogard G", product of Lion Corp), o-[2-hydroxy-3-(trimethylammonio)propyl] guar gum chloride (e.g., "RHABALL gum CG-M", "RHABALL gum CG-6L", "RHABALL gum CG-M7" and "RHABALL gum CG-M8M", each product of Dainippon Sumitomo Pharma, "Jaguar C-13S", "Jaguar C-14S", "Jaguar C-17", "Jaguar C-210", "Jaguar C-162", and "HI-CARE 1000", each product of Rhodia), cationic dextran, methyl cellulose (e.g., "METOLOSE SM", product of Shin-Etsu Chemical), ethyl cellulose (e.g., "Brilliance 515", product of Gattefosse), hydroxyethyl cellulose (e.g., "CELLOSIZE QP4400H" and "CELLOSIZE QP52000H", each product of The Dow Chemical Company, "SE-600" and "SE-850", each product of Daicel Chemical Industries), hydroxypropyl cellulose (e.g., "Nisso HPC-H" and "Nisso HPC-M", each product of Nippon Soda), hydroxypropylmethyl cellulose (e.g., "METOLOSE 60SH series", "METOLOSE 65SH series", and "METOLOSE 90SH series", each product of Shin-Etsu Chemical), pullulan (e.g., "Pullulan PF-20" and "Pullulan PI-20", each product of Hayashibara Group), fatty acid esters of pullulan, xanthan gum (e.g., "ECHO gum", product of Dainippon Sumitomo Pharma), and hydroxypropyl xanthan gum (e.g., "RHABALL gum EX", product of Dainippon Sumitomo Pharma).

These natural polymers and synthetic polymers can be used either singly or in combination. The content of it (or them) in the hair frizziness reducing agent of the present invention is preferably from 0.01 to 20 mass%, more preferably from 0.02 to 10 mass%, even more preferably from 0.05 to 5 mass%.

<Hair growing/hair nourishing component>

The hair frizziness reducing agent of the present invention contains a hair growing/hair nourishing component as defined above. The agent can have an improved hair frizziness reducing effect by containing the hair growing/hair nourishing component. Examples of such a hair growing/hair nourishing component include blood circulation enhancers for enhancing blood circulation of scalp to promote hair growth and follicle activators for activating follicles to promote hair growth. They also include anti-androgen agents and potassium ion channel openers.

Examples of the blood circulation enhancers include carbon dioxide, acetylcholine, carpronium chloride, spironolactone, vitamin $B_6$ hydrochloride, γ-oryzanol, circuletin, cromakalim, cepharanthine, nicorandil, vitamin Es (e.g., DL-α-tocopherol, D-α-tocopherol, DL-α-tocopherol acetate, D-α-tocopherol acetate), nicotinic acid derivatives (e.g., nicotinic acid, DL-α-tocopherol nicotinate, nicotinic acid amide, and benzyl nicotinate), pinacidil, minoxidil, phthalides, diisopropylamine dichloroacetate, ginseng extract, Swertia japonica extract, iodized garlic extract, ginkgo extract, cinchona extract, Acorus calamus root extract, Sophora extract, bitter orange peel extract, Swertia Japonica extract, Capsicum tincture, Citrus junos fruit extract, Cantharides tincture, and ginger tincture. Of these, vitamin Es, nicotinic acid derivatives, diisopropylamine dichloroacetate, Swertia japonica extract, and Capsicum tincture are preferred.

Examples of the follicle activators include flavanonol derivatives (e.g., trans-3,4'-dimethylflavanonol), pantothenic acid derivatives (e.g., pantothenic acid, pantothenates, panthenol, pantothenyl ethyl ether), N-acylamino acid, pentadecanoic acid glyceride, 6-benzylaminopurine, procyanidine, adenosine derivatives (e.g., adenosine, adenosine-5'-phosphoric acid, and salts of adenosine-5'-phosphoric acid), potassium aspartate, sensitive dye 301, biotin, sodium mononitroguayacol, N-acetyl-L-methionine, Stephania cepharantha root extract, ginseng extracts (e.g., Panax japonicus rhizome and Panax ginseng extract), grape extract, apple extract, yeast extract, garlic component, pearl protein extract, placenta extract, jujube extract, and royal jelly extract. Of these, flavanonol derivatives, pantothenic acid derivatives, N-acylamino acid, pentadecanoic acid glyceride, 6-benzylaminopurine, procyanidine, biotin, sodium mononitroguayacol,

Stephania cepharantha root extract, placenta extract, and royal jelly extract are preferred, with flavanonol derivatives, pantothenic acid derivatives, N-acylamino acids, pentadecanoic acid glyceride, 6-benzylaminopurine, and procyanidine being more preferred and flavanonol derivatives being even more preferred.

Examples of the anti-androgen agents include cyproterone acetate, 11$\alpha$-hydroxyprogesteron, flutamide, 3-deoxyadenosine, chlormadinone acetate, ethinyl estradiol, spironolactone, epitesterone, finasteride, aloe, Zanthoxylum fruit, clove extract, Cuachalalate extract, and Panax ginseng extract.

Examples of the potassium channel opener include minoxidil, chromakalim, diazoxide and derivatives thereof and pinacidil.

The content of the hair growing/hair nourishing component added to the hair frizziness reducing agent is preferably from 0.001 to 10 mass%, more preferably from 0.01 to 5 mass%, each in the total composition, from the standpoint of providing a sufficient hair growing effect and suppressing irritation.

<Medicinally effective component>

The hair frizziness reducing agent of the present invention can contain, in addition to the above hair growing/hair nourishing components, various medicinally active components such as antibacterial agents, anti-inflammatory agents, humectants, keratolytic agents, antiseborrheics, topical stimulants, antioxidants, and vitamins.

Examples of the antibacterial agent include isopropyl methyl phenol, benzalkonium chloride, octopirox, photosensitive pigment No. 101, photosensitive pigment No. 201, chlorohexidine, salicylic acid, zinc pyrithione, potassium sorbate, hinokitiol and phenol. Of these, isopropyl methyl phenol, benzalkonium chloride, piroctone olamine, zinc pyrithione and hinokitiol are preferred.

Examples of the anti-inflammatory agents include glycyrrhiza extract, glycyrrhizic acid and derivatives thereof, glycyrrhetinic acid and derivatives thereof, lipophilic glycyrrhetinic acid derivatives, azulene, guaiazulene, antihistaminic agents such as diphenhydramine, hydrocortisone acetate, prednisolone, Scutellaria root extract, camomile extracts, Sasa veitchii extract, white birch extract, Malva sylvestris extract, peach leaf extract, Achillea millefolium extract, platycodon root extract, loquat leaf extract, and linden extract. Of these, glycyrrhiza extract, glycyrrhizic acid and derivatives thereof, glycyrrhetinic acid and derivatives thereof, azulene, guaiazulene, Scutellaria root extract, camomile extracts, Sasa veitchii extract, white birch extract, Malva sylvestris extract, peach leaf extract, and Achillea millefolium extract are preferred.

Examples of the humectant include Hypericum erectum extract, oat extract, platycodon fluidextract, soluble collagen, glycerin, chondroitin sulfate, tuberose polysaccharides, propylene glycol, Cordyceps sinensis extract, Plectranthus japonicus extract, Eucalyptus extract, barley extract, orange extract, grape extract, seaweed extract, Moutan bark extract, Rehmannia glutinosa extract, duke extract, Rosa rugosa extract, Mountan bark extract, and Coix lacryma-jobi extract. Of these, Hypericum erectum extract, oat extract, glycerin, tuberose polysaccharides, Cordyceps sinensis extract, Plectranthus japonicus extract, barley extract, grape extract, propylene glycol, platycodon fluidextract, and Coix lacryma-jobi extract are preferred.

Examples of the keratolytic agent include aspirin.

Examples of the antiseborrheic include sulfur, lecithin, Polygoni multiflori radix extract and tioxolone.

Examples of the topical stimulant include camphor, nonylic acid vanillylamide, Holland mustard extract, zanthoxylum fruit extract, peppermint oil, 1-menthol and horseradish extract. Of these, camphor and 1-menthol are preferred.

Examples of the antioxidant include tea extract, green tea extract, rose fruit extract, Engelhardtia chrysolepis extract, vitamin C and derivatives thereof, erythorbic acid, propyl gallate, and dibutylhydroxytoluene.

The total amount of these medicinally effective components in the whole composition is preferably from 0.001 to 20 mass%, more preferably from 0.01 to 8 mass%.

<Conditioning component>

The hair frizziness reducing agent of the present invention may contain a conditioning component suited for application to the hair. The conditioning component is usually a polymer or oil soluble or dispersible in the hair frizziness reducing agent. When the conditioning component is added, the content of it is from 0.01 to 30 mass%, preferably from 0.1 to 20 mass%, more preferably from 0.1 to 10 mass% in the total composition.

Examples of the preferable conditioning component to be incorporated in the hair frizziness reducing agent include cationic polymers, silicones, and organic conditioning oils, and combinations thereof. In addition, a conditioning agent that forms dispersion liquid particles in a water-based surfactant can also be incorporated.

The term "cationic polymer" means a polymer having a cationic group or a group ionized into a cationic group and it includes an amphoteric polymer which will be cationic as a whole. It also includes a cationic polymer used as the above thickening agent. Examples of the cationic polymer include aqueous solutions having, on the side chain of their polymer chain, an amino group or an ammonium group, or having, as a structural unit thereof, a diallyl quaternary

ammonium salt, such as cationic cellulose derivatives, cationic starches, cationic guar gum derivatives, polymers or copolymers of a diallyl quaternary ammonium salt, and quaternized polyvinylpyrrolidone derivatives. Of these, polymers having, as a structural unit thereof, a diallyl quaternary ammonium salt, quaternized polyvinylpyrrolidone derivatives, and cationic cellulose derivatives are preferred from the standpoint of their effects upon use such as softness, smoothness, and easy finger combability, effects upon drying such as manageability and moisture retention, and stability of the resulting hair frizziness reducing agent, with polymers or copolymers of a diallyl quaternary ammonium salt and cationic cellulose derivatives being more preferred.

Specific examples of the polymers or copolymers of a diallyl quaternary ammonium salt include dimethyl diallyl ammonium chloride polymers (Polyquaternium-6, e.g., "Merquat 100", product of Nalco), dimethyl diallyl ammonium chloride/acrylic acid copolymers (Polyquaternium-22, e.g., "Merquat 280" and "Merquat 295", each product of Nalco), and dimethyl diallyl ammonium chloride/acrylic acid amide copolymers (Polyquaternium-7, e.g., "Merquat 550", product of Nalco).

Specific examples of the quaternized polyvinylpyrrolidone derivatives include quaternary ammonium salts available from a vinylpyrrolidone (VP)/dimethylaminoethyl methacrylate copolymer and diethyl sulfate (Polyquaternium-11, e.g., "Gafquat 734", "Gafquat 755", and "Gafquat 755N", each product of ISP Japan).

Specific examples of the cationic cellulose derivatives include polymers of a quaternary ammonium salt available by adding glycidyl trimethylammonium chloride to hydroxyethyl cellulose (Polyquaternium-10, e.g., "Rheoguard G" and "Rheoguard GP" (product of Lion Corp), and "Polymer JR-125", "Polymer JR-400", "Polymer JR-30M", "Polymer LR-400", and "Polymer LR-30M" (each, product of Amerchol)), and hydroxyethyl cellulose/dimethyl diallyl ammonium chloride copolymers (Polyquaternium-4, e.g., "Celquat H-100" and "Celquat L-200" (each, product of National Starch and Chemical).

Silicones

The following are silicones usable in the invention.

(1) Dimethylpolysiloxane

$$R^2(CH_3)_2SiO-[(CH_3)_2SiO]_m-Si(CH_3)_2R^2$$

(wherein, $R^2$ represents a methyl or hydroxyl group and m represents a number from 1 to 20,000).

Dispersed particles of dimethylpolysiloxane have an average particle size of preferably less than 100 μm, more preferably 50 μm or less, even more preferably 4 μm or less, even more preferably 2 μm or less. The average particle size is, on the other hand, preferably 0.1 μm or greater from the standpoint of feeling upon use and conditioning effect.

As such a dimethylpolysiloxane, usable are those commercially available as "Silicone CF2450", product of Dow Corning Toray containing 60 mass% of a dimethylpolysiloxane oil of the formula (10) in which m stands for from 300 to 6,500 and having an average particle size of 0.8 μm; and "Silicone CF2460", product of Dow Corning Toray containing 50 mass% of a dimethylpolysiloxane oil of the formula (10) in which m stands for from 300 to 6,500 and having an average particle size of 50 μm.

(2) Amino-modified silicones

Various amino-modified silicones are usable. Of these, amino-modified silicones having an average molecular weight of from about 3,000 to 100,000 and described under the name of Amodimethicone in CTFA Dictionary (Cosmetic Ingredient Dictionary/USA), Third Edition are preferred. Examples of the commercially available product thereof include "SM 8704C" (product of Dow Corning Toray), "DC 929" (product of Dow Corning), "KT 1989" (product of GE Toshiba Silicone), and "8500 Conditioning Agent", "DOW CORNING TORAY SS-3588", and "DOW CORNING TORAY SILSTYLE 104" (each, product of Dow Corning Toray).

(3) Other silicones

Examples of silicones other than the above ones include polyether modified silicones, methylphenylpolysiloxane, fatty acid modified silicones, alcohol modified silicones, alkoxy modified silicones, epoxy modified silicones, fluorine modified silicones, cyclic silicones, and alkyl-modified silicones.

Organic conditioning oils

Examples of the organic conditioning oils include hydrocarbon oils, polyolefins, and fatty acid esters.

Examples of the hydrocarbon oil include hydrocarbon oils such as cyclic hydrocarbons, linear aliphatic hydrocarbons (saturated or unsaturated), and branched aliphatic hydrocarbons (saturated or unsaturated), each having at least 10 carbon atoms, and polymers or mixtures of these hydrocarbon oils. The linear hydrocarbon oils have preferably from 12 to 19 carbon atoms, while the branched hydrocarbon oils contain a hydrocarbon polymer and have usually carbon atoms exceeding 19.

As the polyolefins, liquid polyolefins are preferred, of which liquid poly-$\alpha$-olefins are preferred and hydrogenated liquid poly-$\alpha$-olefins are more preferred. The polyolefin usable here is prepared by polymerizing a $C_4$ to $C_{14}$ olefin monomer, preferably from $C_6$ to $C_{12}$ olefin monomer.

Examples of the aliphatic esters include esters (such as monoesters, polyol esters, or di- or tri-carboxylic acid esters) having a hydrocarbon chain derived, for example, from a fatty acid and an alcohol. The hydrocarbon group of these aliphatic esters may further have another compatible functional portion such as amide group, alkoxy group or polyoxyalkylene group or may be covalently bonded thereto. Specific examples of the preferred aliphatic ester include isopropyl myristate, and octyldodecyl myristate.

<Polar solvent>

The hair frizziness reducing agent of the present invention may contain, in addition to the above conditioning component, a polar oil component capable of solubilizing the effective components to promote penetration of them into the scalp and at the same time, improving the feel and stability. Examples of the polar oil component include higher alcohols and fatty acid glycerides. The higher alcohols include lauryl alcohol, stearyl alcohol, oleyl alcohol, isostearyl alcohol, and cetostearyl alcohol. The fatty acid glycerides include monoglycerides, diglycerides, and triglycerides of a fatty acid having from 8 to 18 carbon atoms. Of these, polar oil components in liquid form at 20°C and having preferably 100 mPa·s or less, more preferably 50 mPa·s or less are preferred from the standpoint of preventing crystallization of the effective components, dissolving an oil film made of a lipid of the scalp, and promoting penetration through the skin.

The content of the polar oil component in the total composition is preferably from 0.01 to 5 mass%, more preferably from 0.1 to 2 mass% from the standpoint of promoting penetration of the effective components and improvement in the feel of the agent.

<Antioxidant>

The hair frizziness reducing agent of the present invention may further contain an antioxidant. Examples of the antioxidant include vitamin C and derivatives thereof, erythorbic acid, propyl gallate, BHT (di-n-butylhydroxytoluene), and BHA (butylhydroxyanisole). These antioxidants are effective for improving the storage stability of the hair frizziness reducing agent, particularly effective for improving the storage stability at high temperatures (for example, 50°C). The content of the antioxidant in the total composition is preferably from 0.01 to 0.5 wt.%, more preferably from 0.05 to 0.3 wt.%.

<Other optional components and form>

The hair frizziness reducing agent of the present invention may contain, in addition to the above components, components ordinarily employed in cosmetic fields, depending on the using purpose. Examples of such optional components include inorganic alkalis, oils or fats, amino acid derivatives, protein derivatives, chelating agents, antiseptics, ultraviolet absorbers, pH regulators, plant extracts other than those described above, and perfumes.

The hair frizziness reducing agent of the present invention is provided preferably in the form effectively applied to the scalp and it is provided usually in liquid form, typically, lotion or hair tonic. With a propellant, it can be used as an aerosol. It can also be provided in the form of a cream, gel, wax, leave-on type hair treatment, hair foam, or hair spray.

<Using method>

The hair frizziness reducing agent of the present invention is presumed to produce a hair frizziness reducing effect by penetrating into the scalp to act on the completion layer (on page 13 of "Illustrated Hair Science" ed. by Toshiaki Yasuda and co-written by Takeo Sudo and Norishige Seta, 1978) of the hair root, and reducing the frizziness of the hair which has grown after application of the agent. The hair frizziness reducing agent of the present invention should therefore be used by leaving it on the scalp after application thereto without washing it away.

It is preferred to leave the hair frizziness reducing agent on the scalp for preferably at least 3 hours, more preferably at least 6 hours, even more preferably at least 8 hours after application of it to the scalp in order to achieve

a sufficient hair frizziness reducing effect. The hair frizziness reducing agent is, on the other hand, left on the scalp for not greater than 72 hours, more preferably not greater than 48 hours, even more preferably not greater than 24 hours after application of it to the scalp in order to keep the scalp clean.

The hair frizziness reducing agent of the present invention is preferably applied to the scalp immediately after shampooing. The scalp immediately after shampooing is wet so that compared with application to the dry scalp before shampooing, the hair frizziness reducing agent penetrates into the scalp more easily and is therefore effective for reducing the hair frizziness.

After shampooing, the scalp returns to be dry gradually with the passage of time so that application of the frizziness reducing agent to the scalp as soon as possible after shampooing is effective. Described specifically, it is desired to apply the hair frizziness reducing agent to the scalp preferably within one hour, more preferably within 30 minutes, even more preferably within 15 minutes after shampooing.

In the present invention, application of the hair frizziness reducing agent immediately after towel drying of the shampooed hair is more effective for reducing the hair frizziness. When a bundle of the hair is wetted with water, an amount of the water retained in the bundle exceeds the weight of the hair itself. By the towel drying of the shampooed hair, excessive water retained in the hair bundle is removed so that the hair frizziness reducing agent applied to the scalp remains on the scalp without being diluted with water and produces an improved hair frizziness reducing effect.

From the above standpoint, towel drying after shampooing is preferred, but from the standpoint of the penetration of the hair frizziness reducing agent to the scalp, it is desired to apply the hair frizziness reducing agent to the scalp as soon as possible after shampooing while the scalp is still wet. It is therefore desired to apply the hair frizziness reducing agent to the scalp within one hour, more preferably within 30 minutes, even more preferably within 15 minutes after shampooing/towel drying.

The hair frizziness reducing agent of the present invention has an improved hair frizziness reducing effect when the scalp is massaged after application of the agent thereto.

Since the hair frizziness reducing agent applied to the scalp spreads uniformly on the scalp by physical massaging of the scalp, it has an improved hair frizziness reducing effect.

Specific examples of the scalp massaging method include patting, rubbing, or tapping of the scalp with a tool that will not hurt the scalp such as comb or brush. Alternatively, the scalp may be patted, rubbed, tapped, or massaged with fingers directly or with gloves for efficient massaging.

The hair frizziness reducing agent of the present invention has an improved hair frizziness reducing effect by setting the using frequency at once or more every two days.

The hair frizziness reducing agent of the present invention is expected to have a higher hair frizziness reducing effect by remaining on the scalp and penetrating further into the scalp. Since a portion of the hair frizziness reducing agent on the scalp is washed away by the sebum or sweat without penetrating into the scalp, an amount of the hair frizziness reducing agent remaining on the scalp decreases with the passage of time. It is therefore possible to improve the continuous hair frizziness reducing effect by increasing the using frequency of the hair frizziness reducing agent.

Described specifically, the hair frizziness reducing agent is applied to the scalp preferably at least once every two days, more preferably at least once a day, more preferably at least twice a day.

The hair frizziness reducing agent of the present invention penetrates into the scalp and acts on the completion layer of the hair root to reduce the frizzy form of the hair that grows after application of the hair frizziness reducing agent. As a result, it produces a hair frizziness reducing effect. Accordingly, the hair frizziness reducing effect can be recognized gradually with the growth of the hair. Described specifically, when the hair of at least 2 or 3 cm grows, the hair frizziness reducing effect starts to be recognized. The hair growth rate is about 0.4 mm/day so that periodic use of the hair frizziness reducing agent for 2 months or more facilitates recognition of the hair frizziness reducing effect.

[Examples]

Various hair frizziness reducing agents were prepared and their hair frizziness reducing effect was evaluated in accordance with a specified method by test subjects 40 years of age or older suffering from hair frizziness increased due to aging.

(Formulation of hair frizziness reducing agent)

Hair frizziness reducing agents are prepared in accordance with Formulations A to U shown in Tables 1 and 2 and they are used for the test in Examples and Comparative Examples using hair frizziness reducing methods. All the numerical values in the tables are proportions of the components in terms of mass%. Any of the hair frizziness reducing agents is adjusted to pH 7.0 with an adequate amount of sodium hydroxide. The viscosity of each hair frizziness reducing agent as measured by a Brookfield viscometer at room temperature (23°C) is also shown in Tables 1 and 2.

[Table 1]

| Component/mass% | Formulations | | | | | | | | | | |
|---|---|---|---|---|---|---|---|---|---|---|---|
| | A | B* | C | D | E | F | G | H | I | J | K* |
| Ethanol | 40 | 92 | 65 | 45 | 40 | 40 | 40 | 40 | 40 | 40 | 40 |
| Acrylic acid/alkyl ($C_{10-30}$) acrylate copolymer | 0.12 | 0.0 | 0.0 | 0.01 | 0.04 | 1.5 | 0.5 | 0.5 | 0.5 | 0.5 | 0.5 |
| Guar hydroxypropyltrimonium chloride | 0.0 | 0.0 | 0.0 | 0.0 | 0.0 | 0.0 | 2.0 | 3.0 | 4.0 | 4.5 | 5.0 |
| 1,3-Butylene glycol | 1.5 | 1.5 | 1.5 | 1.5 | 1.5 | 1.5 | 1.5 | 1.5 | 1.5 | 1.5 | 1.5 |
| Propylene glycol | 0.5 | 0.5 | 0.5 | 0.5 | 0.5 | 0.5 | 0.5 | 0.5 | 0.5 | 0.5 | 0.5 |
| Betaine | 0.1 | 0.1 | 0.1 | 0.1 | 0.1 | 0.1 | 0.1 | 0.1 | 0.1 | 0.1 | 0.1 |
| Dipotassium glycyrrhizinate | 0.08 | 0.08 | 0.08 | 0.08 | 0.08 | 0.08 | 0.08 | 0.08 | 0.08 | 0.08 | 0.08 |
| Gardenia Florida extract | 2.0 | 2.0 | 2.0 | 2.0 | 2.0 | 2.0 | 2.0 | 2.0 | 2.0 | 2.0 | 2.0 |
| Eucalyptus extract | 2.0 | 2.0 | 2.0 | 2.0 | 2.0 | 2.0 | 2.0 | 2.0 | 2.0 | 2.0 | 2.0 |
| Glycyrrhiza extract | 0.5 | 0.5 | 0.5 | 0.5 | 0.5 | 0.5 | 0.5 | 0.5 | 0.5 | 0.5 | 0.5 |
| Cymen-5-ol | 0.1 | 0.1 | 0.1 | 0.1 | 0.1 | 0.1 | 0.1 | 0.1 | 0.1 | 0.1 | 0.1 |
| Menthol | 0.2 | 0.2 | 0.2 | 0.2 | 0.2 | 0.2 | 0.2 | 0.2 | 0.2 | 0.2 | 0.2 |
| Perfume | 0.05 | 0.05 | 0.05 | 0.05 | 0.05 | 0.05 | 0.05 | 0.05 | 0.05 | 0.05 | 0.05 |
| Water | Balance | Balance | Balance | Balance | Balance | Balance | Balance | Balance | Balance | Balance | Balance |
| Viscosity (mPa·s) | 100 | <1 | 2 | 3 | 10 | 1,000 | 10,000 | 50,000 | 80,000 | 90,000 | 100,000 |
| *Comparative Compositions | | | | | | | | | | | |

[Table 2]

| Component/mass% | Formulations | | | | | | | | | |
|---|---|---|---|---|---|---|---|---|---|---|
| | L | M | N | O | P | Q | R | S | T | U |
| Ethanol | 40 | 40 | 40 | 40 | 40 | 40 | 40 | 40 | 40 | 40 |
| Acrylic acid/alkyl ($C_{10-30}$) acrylate copolymer | 0.12 | 0.12 | 0.12 | 0.12 | 0.12 | 0.12 | 0.12 | 0.12 | 0.12 | 0.12 |
| Minoxidil | 1.0 | 0.0 | 0.0 | 0.0 | 0.0 | 0.0 | 0.0 | 0.0 | 0.0 | 0.0 |
| Swertia japonica extract | 0.0 | 1.3 | 0.0 | 0.0 | 0.0 | 0.0 | 0.0 | 0.0 | 0.0 | 0.0 |
| Ginkgo extract | 0.0 | 0.0 | 1.0 | 0.0 | 0.0 | 0.0 | 0.0 | 0.0 | 0.0 | 0.0 |
| Tocopherol acetate | 0.0 | 0.0 | 0.5 | 0.0 | 0.0 | 0.0 | 0.0 | 0.0 | 0.0 | 0.0 |
| Capronium chloride | 0.0 | 0.0 | 0.0 | 1.0 | 0.0 | 0.0 | 0.0 | 0.0 | 0.0 | 0.0 |
| Niacinamide | 0.0 | 0.0 | 0.0 | 0.0 | 0.1 | 0.0 | 0.0 | 0.0 | 0.0 | 0.0 |
| Trans-3,4'-dimethylflavanonone | 0.0 | 0.0 | 0.0 | 0.0 | 0.0 | 0.1 | 0.0 | 0.0 | 0.0 | 0.0 |
| Adenosine | 0.0 | 0.0 | 0.0 | 0.0 | 0.0 | 0.0 | 0.5 | 0.0 | 0.0 | 0.0 |
| Pentadecanoic acid glyceride | 0.0 | 0.0 | 0.0 | 0.0 | 0.0 | 0.0 | 0.0 | 2.0 | 0.0 | 0.0 |
| 6-Benzylaminopurine | 0.0 | 0.0 | 0.0 | 0.0 | 0.0 | 0.0 | 0.0 | 0.0 | 0.5 | 0.0 |
| Polygoni multiflori radix extract | 0.0 | 0.0 | 0.0 | 0.0 | 0.0 | 0.0 | 0.0 | 0.0 | 0.0 | 2.0 |
| 1,3-Butylene glycol | 1.5 | 1.5 | 1.5 | 1.5 | 1.5 | 1.5 | 1.5 | 1.5 | 1.5 | 1.5 |
| Propylene glycol | 0.5 | 0.5 | 0.5 | 0.5 | 0.5 | 0.5 | 0.5 | 0.5 | 0.5 | 0.5 |
| Betaine | 0.1 | 0.1 | 0.1 | 0.1 | 0.1 | 0.1 | 0.1 | 0.1 | 0.1 | 0.1 |
| Dipotassium glycyrrhizinate | 0.08 | 0.08 | 0.08 | 0.08 | 0.08 | 0.08 | 0.08 | 0.08 | 0.08 | 0.08 |
| Gardenia Florida extract | 2.0 | 2.0 | 2.0 | 2.0 | 2.0 | 2.0 | 2.0 | 2.0 | 2.0 | 2.0 |
| Eucalyptus extract | 2.0 | 2.0 | 2.0 | 2.0 | 2.0 | 2.0 | 2.0 | 2.0 | 2.0 | 2.0 |
| Glycyrrhiza extract | 0.5 | 0.5 | 0.5 | 0.5 | 0.5 | 0.5 | 0.5 | 0.5 | 0.5 | 0.5 |
| Cymen-5-ol | 0.1 | 0.1 | 0.1 | 0.1 | 0.1 | 0.1 | 0.1 | 0.1 | 0.1 | 0.1 |
| Menthol | 0.2 | 0.2 | 0.2 | 0.2 | 0.2 | 0.2 | 0.2 | 0.2 | 0.2 | 0.2 |
| Perfume | 0.05 | 0.05 | 0.05 | 0.05 | 0.05 | 0.05 | 0.05 | 0.05 | 0.05 | 0.05 |
| Water | Balance | Balance | Balance | Balance | Balance | Balance | Balance | Balance | Balance | Balance |

(continued)

| Component/mass% | Formulations | | | | | | | | | |
|---|---|---|---|---|---|---|---|---|---|---|
| | L | M | N | O | P | Q | R | S | T | U |
| Viscosity (mPa·s) | 100 | 100 | 100 | 100 | 100 | 100 | 100 | 100 | 100 | 100 |

(Using method of hair frizziness reducing agent)

The hair frizziness reducing agent should be used in accordance with the following procedures 1 to 6. It is to be noted that treatment may be performed without some of the procedures.

Procedure 1: Shampooing before application of a hair frizziness reducing agent to scalp

In the test of Examples, when shampooing is performed prior to the application of a hair frizziness reducing agent to the scalp, a shampooing method of test subjects is not particularly limited, but the hair is shampooed with an ordinarily used shampoo and then the shampoo is rinsed with water or warm water having a proper temperature. After shampooing, a rinse, conditioner, or a hair treatment may be used, followed by rinsing.

Procedure 2: Towel-drying after shampooing

The term "towel drying" means removal of water from the hair, which has become wet by shampooing, with a towel. Since towel drying is performed usually for about 1 minute and, at longest, for about 15 minutes, the hair is not dried completely even by the towel drying. This means that the hair and scalp after towel drying is usually half dry but still wet.

In the test of Examples, upon towel drying, in order to avoid excessive towel drying prior to the application of the hair frizziness reducing agent to the scalp, the towel drying time is limited to 15 minutes at longest and the test subjects are asked to dry their hair and scalp into a half dry and still wet state.

Procedure 3: Leaving the hair during a time after shampooing/towel drying to the application of a hair frizziness reducing agent

When hair is shampooed before application of a hair frizziness reducing agent to the scalp, a leaving time until the application of the hair frizziness reducing agent is from about 0 minute to about one hour at longest. In the test of Examples, when shampooing and towel drying are performed, the test subjects are asked to leave the hair for about 6 minutes (0.1 hour) until the application of the hair frizziness reducing agent.

In the test of Examples/Comparative Examples, when hair is not shampooed prior to the application of the hair frizziness reducing agent to the scalp, the test subjects are asked to control the leaving time between shampooing to the application of the hair frizziness reducing agent to 8 hours or more.

Procedure 4: Application of hair frizziness reducing agent to scalp

In the test of Examples/Comparative Examples, the test subjects are asked to part their hair at several sites, that is, top site, right and left temporal sites, and occipital site of the head and apply the hair frizziness reducing agent directly to the scalp at the root of these sites. A total amount of the hair frizziness reducing agent applied to the entire head is about 2.5 mL/once.

Procedure 5: Rinsing (washing-away) of hair frizziness reducing agent applied to the scalp

In the test of Comparative Example, when the hair frizziness reducing agent applied to the scalp is washed away, the test subjects are asked to wash away the hair frizziness reducing agent with water or warm water having a proper temperature within 5 minutes after application of the agent.

Procedure 6: Scalp massage after application of hair frizziness reducing agent to scalp.

In the test of Examples, when scalp massage is performed after application of the hair frizziness reducing agent to the scalp, the test subjects are asked to carry out an operation of scalp massaging, that is, patting, rubbing, tapping, massaging or the like with fingers for from 1 to 10 minutes to spread the agent across the scalp.

The using methods of the hair frizziness reducing agent in Examples/Comparative Examples in accordance with the above using procedures of the hair frizziness reducing agent are shown in Tables 3 and 4. the tables also include using frequency/using duration of the hair frizziness reducing agent in each using method.

[Table 3]

| Using procedures | Method A | Method B* | Method C | Method D | Method E |
|---|---|---|---|---|---|
| 1. Shampooing before application of hair frizziness reducing agent | Not performed | Not performed | Performed | Performed | Not performed |
| 2. Towel drying after shampooing | Not performed | Not performed | Not performed | Performed | Not performed |
| 3. Leaving time from shampooing/towel drying to application of the agent | 8 hr or more | 8 hr or more | 0.1 hr | 0.1 hr | 8 hr or more |
| 4. Application of hair frizziness reducing agent to scalp | Performed | Performed | Performed | Performed | Performed |
| 5. Rinsing after application of the agent | Not performed | Performed | Not performed | Not performed | Not performed |
| 6. Scalp massaging | Not performed | Not performed | Not performed | Not performed | Performed |
| Using frequency (times/day) of hair frizziness reducing agent | 2 | 2 | 2 | 2 | 2 |
| Using duration (month) of hair frizziness reducing agent | 6 | 6 | 6 | 6 | 6 |
| *Comparative Method | | | | | |

[Table 4]

| Using procedures | Method F | Method G | Method H | Method I | Method J |
|---|---|---|---|---|---|
| 1. Shampooing before application of hair frizziness reducing agent | Not performed | Not performed | Not performed | Not performed | Not performed |
| 2. Towel drying after shampooing | Not performed | Not performed | Not performed | Not performed | Not performed |
| 3. Leaving time from shampooing/towel drying to application of the agent | 8 hr or more | 8 hr or more | 8 hr or more | 8 hr or more | 8 hr or more |
| 4. Application of hair frizziness reducing agent to scalp | Performed | Performed | Performed | Performed | Performed |
| 5. Rinsing after application of the agent | Not performed | Not performed | Not performed | Not performed | Not performed |
| 6. Scalp massaging | Not performed | Not performed | Not performed | Not performed | Not performed |
| Using frequency (times/day) of hair frizziness reducing agent | 1 | 0.5 | 0.2 | 2 | 2 |
| Using duration (month) of hair frizziness reducing agent | 6 | 6 | 6 | 2 | 1 |

Using method A of Table 3 is taken for an example. Shampooing of Procedure 1 is omitted. This means that towel drying in Procedure 2 is not necessary. In this case, at least 8 hours have passed since previous shampooing so that the leaving time in Procedure 3 is "8 hours or more". After application of the hair frizziness reducing agent in Procedure 4, rinsing of Procedure 5 and scalp massaging of Procedure 6 are not performed. In this case, the hair frizziness reducing agent is periodically used twice a day for 6 months.

(Evaluation method of hair frizziness reducing effect)

Evaluations A to C shown below are performed and hair frizziness reducing effect is evaluated by comparing the values before and after hair frizziness reducing treatment. At the same time, dripping property (Evaluation D) upon application of the hair frizziness reducing agent and spreadability (Evaluation E) of the hair frizziness reducing agent on the scalp are evaluated.

Evaluation A: Evaluation of the shape of hair 3 cm from the root

Hair 3 cm from the root thereof is evaluated based on the following five-grade visual evaluation system: 1: strongly frizzy hair, 2: frizzy hair, 3: somewhat frizzy hair, 4: slightly frizzy hair, and 5: straight hair. The frizziness reducing effect of the agent is evaluated by comparing the hair frizziness before and after application of the agent. In order to observe the shape of the hair root, the hair is parted at the center of the head top with a comb and the shape of the hair at about 3 cm from the root at the division is visually evaluated. A difference in the evaluation score between before and after the application of the hair frizziness reducing agent is determined to evaluate the hair frizziness reducing effect. For example, when the evaluation score before use is 1 and that after use is 4, the frizziness reducing effect determined from the following equation:

(score after use) - (score before use) = 4-1 is 3.

Evaluation B: Evaluation of hair shape of the entire head

An amount of frizzy hair of the entire head is evaluated based on the following five-grade visual evaluation system: 1: very large, 2: somewhat large, 3: somewhat small, 4: small, and 5: scarce and a frizziness reducing effect is evaluated by comparing the amount before and after application of the hair frizziness reducing agent. The frizziness reducing effect on the entire head is evaluated by determining a difference in the evaluation score before and after application of the hair frizziness reducing agent. For example, when the score before application is 1 and the score after application is 4, the frizziness reducing effect determined from the following equation: (score after use)-(score before use) =4-1 is 3.

Evaluation C: Evaluation of hair luster

The hair luster is evaluated based on the following five-grade visual evaluation system: 1: having no luster, 2: having slight luster, 3: having moderate luster, 4: having somewhat luster, and 5: having adequate luster and a luster improving effect is evaluated by comparing it before and after application of the hair frizziness reducing agent. The hair luster improving effect is evaluated by determining a difference in the evaluation score before and after application of the hair frizziness reducing agent. For example, when the score before use is 1 and the score after use is 4, the luster improving effect determined based on the following equation: (score after use) - (score before use) =4-1 is 3. The evaluation of hair luster varies greatly depending on illumination conditions so that evaluation of luster is performed under predetermined illumination conditions.

Evaluation D: Evaluation of dripping property upon application of hair frizziness reducing agent

Dripping property upon application of the hair frizziness reducing agent is evaluated based on the following four-grade evaluation system: 1: dripping occurs, 2: somewhat dripping occurs, 3: almost no dripping occurs, 4: no dripping occurs.

Evaluation E: Spreadability of hair frizziness reducing agent on scalp upon application

Spreadability of the hair frizziness reducing agent on scalp when the agent is applied thereto is evaluated based on the following four-grade evaluation system: 1: the agent cannot be applied uniformly due to very poor spreadability, 2: the agent is not easily applied uniformly due to somewhat poor spreadability, 3: the agent is easily applied uniformly due to somewhat good spreadability, 4: the agent can be applied uniformly due to good spreadability.

(Test subjects)

The above test subjects of Evaluations A to E are selected at random from Japanese females as old as 40 years old or greater. The increasing degree of hair frizziness due to aging differs greatly among individuals so that the degree of hair frizziness reducing effect also varies greatly among individuals. In order to evaluate a difference in the hair frizziness reducing effect under similar conditions as much as possible, the test subjects are selected from the Japanese females having severe hair frizziness due to aging. Described specifically, test subjects are selected at random from the Japanese females whose evaluation scores before application in Evaluations A to C are each 1.
The test of Examples/Comparative Examples is made for at least five test subjects.

(Evaluators of each evaluation)

A plurality of beauticians and researches engaged in development of hair care products carries out above Evaluations A to C objectively. The results described in Examples and Comparative Examples are each comprehensive evaluation results made by the evaluators for the test subjects.
With regard to the above Evaluations D to E, on the other hand, the test subjects are asked to subjectively evaluate the hair frizziness reducing agent used by them in practice and at the same time, a plurality of beauticians and researches engaged in development of hair care products are asked to make an objective evaluation. The results described in Examples and Comparative Examples are each comprehensive evaluation results made by the test subjects and the evaluators.

Result 1 (Influence of viscosity on hair frizziness reducing agent)

The hair frizziness reducing agents used in Examples 1 to 9 and Comparative Examples 1 and 2, the hair frizziness reducing method, evaluation results of hair frizziness reducing effect, and evaluation results of dripping property and spreadability of the agent applied to the scalp are shown in Tables 5 and 6.

[Table 5]

| | Ex. 1 | Ex. 2 | Ex. 3 | Ex. 4 | Ex. 5 | Ex. 6 |
|---|---|---|---|---|---|---|
| Hair frizziness reducing agent used for evaluation | Formulation A | Formulation C | Formulation D | Formulation E | Formulation F | Formulation G |
| Hair frizziness reducing method | Method A | Method A | Method A | Method A | Method A | Method A |
| Evaluation A (shape of hair root) | 3 | 2 | 3 | 3 | 3 | 3 |
| Evaluation B (entire shape) | 3 | 2 | 3 | 3 | 3 | 3 |
| Evaluation C (luster) | 3 | 2 | 3 | 3 | 3 | 3 |
| Evaluation D (dripping property) | 3 | 2 | 2 | 3 | 4 | 4 |
| Evaluation E (spreadability of agent) | 4 | 4 | 4 | 4 | 4 | 3 |

[Table 6]

| | Ex. 7 | Ex. 8 | Ex.9 | Comp. Ex. 1 | Comp. Ex. 2 |
|---|---|---|---|---|---|
| Hair frizziness reducing agent used for evaluation | Formulation H | Formulation I | Formulation J | Formulation B | Formulation K |

(continued)

| | Ex. 7 | Ex. 8 | Ex.9 | Comp. Ex. 1 | Comp. Ex. 2 |
|---|---|---|---|---|---|
| Hair frizziness reducing method | Method A | Method A | Method A | Method A | Method A |
| Evaluation A (shape of hair root) | 2 | 2 | 2 | 1 | 1 |
| Evaluation B (entire shape) | 3 | 3 | 2 | 1 | 1 |
| Evaluation C (luster) | 2 | 2 | 2 | 1 | 1 |
| Evaluation D (dripping property) | 4 | 4 | 4 | 1 | 4 |
| Evaluation E (spreadability of agent) | 3 | 3 | 3 | 2 | 1 |

As is apparent from Tables 5 and 6, the hair frizziness reducing agents of Examples 1 to 9 having a viscosity within a range of from 1 to 90,000 mPa·s show good results in dripping property and spreadability and produce a good hair frizziness reducing effect with an evaluation score greater by at least 2. The hair frizziness reducing agent of Comparative Example 1, on the other hand, has a too low viscosity so that dripping occurs when the agent is applied to the scalp and as a result, its hair frizziness reducing effect is low. The hair frizziness reducing agent of Comparative Example 2 has an excessively high viscosity so that it shows poor spreadability when the agent is applied to the scalp and as a result, its hair frizziness reducing effect is low.

The results of Tables 5 and 6 have therefore revealed that the viscosity of the hair frizziness reducing agent within a range of from 1 to 90,000 mPa·s is appropriate.

Result 2 (Influence of rinsing after application of the agent)

The hair frizziness reducing agents used in Examples 1 and 5 and Comparative Examples 3 and 4, the hair frizziness reducing methods, and evaluation results of the hair frizziness reducing effect are shown in Table 7.

[Table 7]

| | Ex. 1 | Ex. 5 | Comp. Ex. 3 | Comp. Ex. 4 |
|---|---|---|---|---|
| Hair aging preventive used for evaluation | Formulation A | Formulation F | Formulation A | Formulation F |
| Hair aging preventing method | Method A | Method A | Method B | Method B |
| Evaluation A (shape of hair root) | 3 | 3 | 0 | 0 |
| Evaluation B (shape of entire hair) | 3 | 3 | 0 | 0 |
| Evaluation C (luster) | 3 | 3 | 0 | 0 |

As is apparent from Table 7, when the hair frizziness reducing agent applied to the scalp is left without washing away as in Examples 1 and 5, the hair frizziness reducing effect is good. On the other hand, when the hair frizziness reducing agent is applied to the scalp and then rinsed and washed away as in Comparative Examples 3 and 4, no hair frizziness reducing effect is observed because the agent does not remain on the scalp.

The results of Table 7 have revealed that it is essential to leave the hair frizziness reducing agent applied to the scalp as is without washing it away in order to achieve a good hair frizziness reducing effect.

Result 3 (Influence of a hair growing/hair nourishing component)

The hair frizziness reducing agents used in Examples 10 to 19, the hair frizziness reducing method, and evaluation results of hair frizziness reducing effect are shown in Table 8.

[Table 8]

| | Examples | | | | | | | | | |
|---|---|---|---|---|---|---|---|---|---|---|
| | 10 | 11 | 12 | 13 | 14 | 15 | 16 | 17 | 18 | 19 |
| Hair frizziness reducing agent used for evaluation | Formulation L | Formulation M | Formulation N | Formulation O | Formulation P | Formulation Q | Formulation R | Formulation S | Formulation T | Formulation U |
| Hair frizziness reducing method | Method A | Method A | Method A | Method A | Method A | Method A | Method A | Method A | Method A | Method A |
| Evaluation A (shape of hair root) | 4 | 4 | 4 | 4 | 4 | 4 | 4 | 4 | 4 | 4 |
| Evaluation B (entire hair shape) | 3 | 4 | 4 | 4 | 3 | 3 | 3 | 4 | 4 | 4 |
| Evaluation C (luster) | 3 | 4 | 3 | 4 | 4 | 3 | 4 | 4 | 3 | 4 |

As is apparent from Table 8, the hair frizziness reducing effect in Examples 10 to 19 wherein the hair frizziness reducing agent containing a hair growing/hair nourishing component is used is better than that achieved in Example 1 wherein the hair frizziness reducing agent is free from a hair growing/hair nourishing component.
Results of Table 8 have revealed that use of the hair frizziness reducing agent containing a hair growing/hair nourishing component improves a hair frizziness reducing effect.

Result 4 (Influence of shampooing/towel drying/massaging)

The hair frizziness reducing agent used in Examples 20 to 22, the hair frizziness reducing methods, and evaluation results of hair frizziness reducing effect are shown in Table 9.

[Table 9]

| | Example 20 | Example 21 | Example 22 |
|---|---|---|---|
| Hair frizziness reducing agent used for evaluation | Formulation A | Formulation A | Formulation A |
| Hair frizziness reducing method | Method C | Method D | Method E |
| Evaluation A (shape of hair root) | 4 | 4 | 4 |
| Evaluation B (entire hair shape) | 3 | 4 | 4 |
| Evaluation C (luster) | 3 | 4 | 3 |

As is apparent from Table 9, the hair frizziness reducing effect of Example 20 wherein the hair frizziness reducing agent is applied to the scalp wetted by shampooing performed 0.1 hour before application is better than that of Example 1 wherein the hair frizziness reducing agent is applied without shampooing before application.
Moreover, the hair frizziness reducing effect of Example 21 wherein the agent is applied to the scalp which has been shampooed and then towel-dried to remove excessive moisture from a hair bundle is even better than that of Example 20 in which towel drying is not performed.
The hair frizziness reducing effect of Example 22 wherein scalp massage is performed after application of the hair frizziness reducing agent to the scalp is even better than that of Example 1 wherein no massage is performed after application.
The results of Examples 20 to 22 in Table 9 have revealed that shampooing prior to the application of the hair frizziness reducing agent, towel drying following the shampooing, and scalp massage after application of the agent improve the hair frizziness reducing effect.

Result 5 (Influence of using frequency/duration of use)

The hair frizziness reducing agent used in Examples 23 to 27, the hair frizziness reducing methods, and evaluation results of hair frizziness reducing effect are shown in Table 10.

[Table 10]

| | Ex. 23 | Ex. 24 | Ex. 25 | Ex. 26 | Ex. 27 |
|---|---|---|---|---|---|
| Hair aging preventive for evaluation | Formulation A | Formulation A | Formulation A | Formulation A | Formulation A |
| Hair aging preventing method | Method F | Method G | Method H | Method I | Method J |
| Evaluation A (shape of hair root) | 3 | 2 | 1 | 3 | 1 |
| Evaluation B (entire hair shape) | 2 | 1 | 1 | 1 | 0 |
| Evaluation C (luster) | 2 | 1 | 1 | 1 | 0 |

As is apparent from Table 10, the hair frizziness reducing effect of Examples 23 to 25 wherein using frequency is lower than that of Example 1 becomes lower with a decrease in using frequency.
The hair frizziness reducing effect recognizable in Examples 26 and 27 wherein the duration of use is shorter than that of Example 1 becomes lower with a decrease in the duration of use. It is however to be noted that since the effect starts to appear from the hair root portion where hair grows, the effect of the agent on the hair root portion is recognized even if the duration of use is short.

The above results have revealed that improvement in the hair frizziness reducing effect can be recognized when the using frequency of the hair frizziness reducing agent is higher and duration of use is longer.

## Claims

1. Use of an agent, which is applied to the scalp as is and left on the scalp without washing away, for reducing the frizziness of the hair which has grown after the application of the agent,
wherein the agent comprises a lower alcohol in an amount of 5 mass% to 80 mass%, water, and a hair growing/nourishing component; and has a viscosity of from 1 to 90,000 mPa*s, measured by a Brookfield viscometer at 23°C;
wherein the lower alcohol is at least one selected from ethanol, 1- propanol, 2-propanol, n-butanol, 2-phenoxyethanol, benzyl alcohol, 2- phenylethanol, 2-benzyloxyethanol, triethylene glycol, 1,3-butylene glycol, propylene glycol, hexylene glycol, and diethylene glycol; and
wherein the hair growing/nourishing component is at least one selected from blood circulation enhancers, follicle activators, anti-androgen agents, and potassium ion channel openers.

2. The use according to claim 1, wherein the agent is applied to the scalp once or more every two days.

3. The use according to claim 1, wherein the agent is periodically applied to the scalp for 2 months or more.

4. The use according to claim 1, wherein the agent is applied to the scalp immediately after shampooing.

5. The use according to claim 1, wherein the agent is applied to the scalp immediately after the shampooing and towel-drying.

6. The use according to claim 1, wherein the agent is applied to the scalp, followed by scalp massaging.

7. Method for reducing the frizziness of hair which has grown after the application of an agent,
wherein the method comprises application of the agent to the scalp as is an leaving it on the scalp without washing away; the agent being periodically applied to the scalp once or more every two days for 2 months or more; and wherein the agent comprises a lower alcohol in an amount of 5 mass% to 80 mass%, water, and a hair growing/nourishing component; and has a viscosity of from 1 to 90,000 mPa*s, measured by a Brookfield viscometer at 23°C;
wherein the lower alcohol is at least one selected from ethanol, 1- propanol, 2-propanol, n-butanol, 2-phenoxyethanol, benzyl alcohol, 2- phenylethanol, 2-benzyloxyethanol, triethylene glycol, 1,3-butylene glycol, propylene glycol, hexylene glycol, and diethylene glycol; and
wherein the hair growing/nourishing component is at least one selected from blood circulation enhancers, follicle activators, anti-androgen agents, and potassium ion channel openers.

8. The method according to claim 7, wherein the agent is applied to the scalp immediately after shampooing.

9. The method according to claim 7, wherein the agent is applied to the scalp immediately after the shampooing and towel-drying.

10. The method according to claim 7, wherein the agent is applied to the scalp, followed by scalp massaging.

## Patentansprüche

1. Verwendung eines Mittels, das, so wie es ist, auf die Kopfhaut aufgetragen wird und auf der Kopfhaut verbleibt, ohne es auszuwaschen, um die Kräuselung des Haares, das nach dem Aufbringen des Mittels gewachsen ist, zu verringern,
wobei das Mittel einen niederen Alkohol in einer Menge von 5 bis 80 Masse-%, Wasser und eine Haarwachstums-/Pflege-Komponente umfasst und eine Viskosität von 1 bis 90.000 mPa·s, gemessen mit einem Brookfield-Viskosimeter bei 23°C, aufweist;
wobei der niedere Alkohol zumindest einer, ausgewählt aus Ethanol, 1-Propanol, 2-Propanol, n-Butanol, 2-Phenoxyethanol, Benzylalkohol, 2-Phenylethanol, 2-Benzyloxyethanol, Triethylenglykol, 1,3-Butylenglykol, Propylenglykol, Hexylenglykol und Diethylenglykol, ist; und
wobei die Haarwachstums-/Pflege-Komponente zumindest eine, ausgewählt aus Blutzirkulationsverbesserern, Follikelaktivatoren, Anti-Androgenmitteln und Kaliumionenkanalöffnern, ist.

2. Verwendung gemäss Anspruch 1, wobei das Mittel jeden zweiten Tag ein- oder mehrmals auf die Kopfhaut aufgetragen wird.

3. Verwendung gemäss Anspruch 1, wobei das Mittel 2 Monate oder länger periodisch auf die Kopfhaut aufgetragen wird.

4. Verwendung gemäss Anspruch 1, wobei das Mittel sofort nach dem Shampoonieren auf die Kopfhaut aufgetragen wird.

5. Verwendung gemäss Anspruch 1, wobei das Mittel sofort nach dem Shampoonieren und Trocknen mit einem Handtuch auf die Kopfhaut aufgetragen wird.

6. Verwendung gemäss Anspruch 1, wobei das Mittel auf die Kopfhaut aufgetragen und die Kopfhaut anschliessend massiert wird.

7. Verfahren zur Verringerung der Kräuselung des Haares, das nach dem Auftragen des Mittels gewachsen ist,
wobei das Verfahren das Aufbringen des Mittels auf die Kopfhaut, so wie sie ist, und Belassen auf der Kopfhaut, ohne es auszuwaschen, umfasst;
das Mittel jeden zweiten Tag ein- oder mehrmals während 2 Monaten oder länger periodisch auf die Kopfhaut aufgetragen wird; und
wobei das Mittel einen niederen Alkohol in einer Menge von 5 bis 80 Masse-%, Wasser und eine Haarwachstums-/Pflege-Komponente umfasst umfasst und eine Viskosität von 1 bis 90.000 mPa·s, gemessen mit einem Brookfield-Viskosimeter bei 23°C, aufweist;
wobei der niedere Alkohol zumindest einer, ausgewählt aus Ethanol, 1-Propanol, 2-Propanol, n-Butanol, 2-Phenoxyethanol, Benzylalkohol, 2-Phenylethanol, 2-Benzyloxyethanol, Triethylenglykol, 1,3-Butylenglykol, Propylenglykol, Hexylenglykol und Diethylenglykol, ist; und
wobei die Haarwachstums-/Pflege-Komponente zumindest eine, ausgewählt aus Blutzirkulationsverbesserern, Follikelaktivatoren, Anti-Androgenmitteln und Kaliumionenkanalöffnern, ist.

8. Verfahren gemäss Anspruch 7, wobei das Mittel sofort nach dem Shampoonieren auf die Kopfhaut aufgetragen wird.

9. Verfahren gemäss Anspruch 7, wobei das Mittel sofort nach dem Shampoonieren und Trocknen mit einem Handtuch auf die Kopfhaut aufgetragen wird.

10. Verfahren gemäss Anspruch 7, wobei das Mittel auf die Kopfhaut aufgetragen und die Kopfhaut anschliessend massiert wird.

**Revendications**

1. Utilisation d'un agent, qui est appliqué au cuir chevelu tel quel et est laissé sur le cuir chevelu sans lavage, pour réduire le bouclage des cheveux qui ont poussé après l'application de l'agent,
dans laquelle l'agent comprend un alcool inférieur en une quantité de 5 % en masse à 80 % en masse, de l'eau et un composant de croissance / nourrissant pour les cheveux ; et a une viscosité de 1 à 90 000 mPa*s, mesurée par un viscosimètre Brookfield à 23°C;
dans laquelle l'alcool inférieur est au moins un sélectionné parmi l'éthanol, 1-propanol, 2-propanol, n-butanol, 2-phénoxyéthanol, alcool de benzyle, 2-phényléthanol, 2-benzyloxyéthanol, triéthylèneglycol, 1,3-butylène glycol, propylène glycol, hexylène glycol et diéthylèneglycol; et
dans laquelle le composant de croissance / nourrissant pour les cheveux est au moins un sélectionné parmi des stimulateurs de circulation sanguine, des activateurs de follicule, des agents anti-androgènes, et des ouvreurs de canal d'ion potassium.

2. Utilisation selon la revendication 1, dans laquelle l'agent est appliqué au cuir chevelu une fois ou plus tous les deux jours.

3. Utilisation selon la revendication 1, dans laquelle l'agent est périodiquement appliqué au cuir chevelu pendant 2

mois ou plus.

**4.** Utilisation selon la revendication 1, dans laquelle l'agent est appliqué au cuir chevelu immédiatement après un shampooing.

**5.** Utilisation selon la revendication 1, dans laquelle l'agent est appliqué au cuir chevelu immédiatement après le shampooing et un séchage à la serviette.

**6.** Utilisation selon la revendication 1, dans laquelle l'agent est appliqué au cuir chevelu, suivi par un massage du cuir chevelu.

**7.** Procédé pour réduire le bouclage des cheveux qui ont poussé après l'application d'un agent,
dans lequel le procédé comprend l'application de l'agent au cuir chevelu tel quel et en le laissant sur le cuir chevelu sans lavage ; l'agent étant périodiquement appliqué au cuir chevelu une fois ou plus tous les deux jours pendant 2 mois ou plus ; et
dans lequel l'agent comprend un alcool inférieur en une quantité de 5 % en masse à 80 % en masse, de l'eau et un composant de croissance / nourrissant pour les cheveux ; et a une viscosité de 1 à 90 000 mPa*s, mesurée par un viscosimètre Brookfield à 23°C;
dans lequel l'alcool inférieur est au moins un sélectionné parmi l'éthanol, 1-propanol, 2-propanol, n-butanol, 2-phénoxyéthanol, alcool de benzyle, 2-phényléthanol, 2-benzyloxyéthanol, triéthylèneglycol, 1,3-butylène glycol, propylène glycol, hexylène glycol et diéthylèneglycol; et
dans lequel le composant de croissance / nourrissant pour les cheveux est au moins un sélectionné à partir de stimulateurs de circulation sanguine, d'activateurs de follicule, d'agents anti-androgènes et d'ouvreurs de canal d'ion potassium.

**8.** Procédé selon la revendication 7, dans lequel l'agent est appliqué au cuir chevelu immédiatement après un shampooing.

**9.** Procédé selon la revendication 7, dans lequel l'agent est appliqué au cuir chevelu immédiatement après le shampooing et un séchage à la serviette.

**10.** Procédé selon la revendication 7, dans lequel l'agent est appliqué au cuir chevelu, suivi par un massage du cuir chevelu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6305942 A
- JP 2000038323 A
- WO 200191705 A
- WO 2005084620 A
- WO 200126604 A


### Non-patent literature cited in the description


- **FRESNO et al.** *Il Farmaco,* 2001, vol. 56, 443-445
- **TAKEO SUDO ; NORISHIGE SETA.** Illustrated Hair Science. 1978, 13